# EUROPEAN PATENT APPLICATION

(11) **EP 4 574 089 A1**
(43) Date of publication of application: **25.06.2025**
(21) Application number: 23853848.2
(22) Date of filing: 10.03.2023
(51) Int. Cl.: A61F 2/01

(54) **DEVICE FOR BLOCKING THROMBUS IN BLOOD VESSEL**

(30) Priority: 17.08.2022 CN 202210985043
(71) Applicant: Shangai Kegang Medical Technology Co., Ltd, Shanghai 201613 (CN)
(72) Inventor: ZHANG, Zhichao, Shanghai 201601 (CN); WANG, Wenzhe, Shanghai 201601 (CN); QIAN, Hengyue, Shanghai 201601 (CN); ZHOU, Zhilong, Shanghai 201601 (CN); MA, Changsheng, Shanghai 201601 (CN); DONG, Jianzeng, Shanghai 201601 (CN)
(74) Representative: Cameron Intellectual Property Ltd
(86) International application number: PCT/CN2023/080751
(87) International publication number: WO 2024/036931

(57) **Abstract**

The present disclosure provides an embolic protection device for intravascular use. A metal wire or strip with an elliptical or flat cross-section is formed in a coaxial multi-turn spiral or vortex line, to be used as an embolic protection device for intravascular implantation. The disclosure optimizes the performance of the embolic protection device for intravascular use, enhancing the ability to intercept thrombi.

## Description

### Field of this disclosure

The present disclosure relates to a medical device, specifically an embolic protection device for intravascular use.

### Background of the disclosure

Technologies mentioned in CN104736102A and the patent US20080183206A1 by BATISTE, STANLEY, which are the only known technologies currently available that use a single wire to construct a vascular embolic protection device.

The basic principle of CN104736102A involves presetting a shape using a superelastic metal wire, which is delivered into the target blood vessel cavity via a hollow needle. Upon insertion, the wire recovers to its preset shape and stabilizes within the vessel to intercept thrombi.

There are two embodiments in the current patents and product information:

One embodiment involves forming a spiral line within the blood vessel, with the axis of the spiral line perpendicular to the direction of blood flow. In this embodiment, if the gap of the spiral line is too small, it can intercept thrombi but will reduce blood flow. If the gap is too large, thrombi will pass through the gap, failing to achieve the purpose of intercepting thrombi.

The other embodiment involves forming a family of spiral lines with a single metal wire within the blood vessel, with the axes of the spiral lines parallel to each other but different, and the axes parallel to the direction of blood flow. This embodiment successfully solves the problem of balancing blood flow and thrombus interception capability. However, to achieve this technical effect, non-coaxial spiral lines are used. The production of coaxial spiral lines is more challenging due to the need for smaller wire diameters to intercept thrombi. However, smaller wire diameter means that the metal wire is more susceptible to deformation by blood flow, thereby losing its intercepting performance. Non-coaxial spiral lines also complicate mold embodiment in production and reduce the yield of heat setting.

The patent by BATISTE, STANLEY uses coaxial spiral lines. However, since its application environment is veins and the target thrombus size is relatively large, it can use thinner metal wires and larger spiral diameters to achieve the desired effect. However, in cases where the blood vessel wall elasticity is strong, the embolic protection device for intravascular use will deform with the pulsation of the blood vessel wall, leading to blood flow fluctuations. Blood flow fluctuations can cause local blood flow turbulence, further increasing the likelihood of thrombus formation.

Therefore, existing technologies need further improvement to reduce blood flow disturbance, intercept smaller thrombi, and simplify processing difficulties.

### Summary of the disclosure

The present disclosure provides a solution to existing challenges in embolic protection devices, including complex manufacturing processes, significant blood flow disturbance, and difficulties intercepting smaller thrombi.

To achieve the above objectives, the present disclosure provides an embolic protection device for intravascular use, using a metal wire or strip with an elliptical or flat cross-section that is formed into an expected shape, which is a coaxial multi-turn spiral or vortex line, to be used as an intravascular embolic protection device. The single wire can become a straight line in a constrained state and will recover to the preset shape in a free state.

Furthermore, the embolic protection device for intravascular use can be deployed in a tubular object and introduced into the target blood vessel through the tubular object. The material's elasticity can recover to the expected shape to achieve the purpose of intercepting thrombi.

The present disclosure also provides an embolic protection device for intravascular use, which includes three parts: an embolic protection device, a connecting ring, and a retract wire. The embolic protection device is a spiral line formed by a flat superelastic metal wire. The connecting ring connects the embolic protection device with the retract wire. The retract wire is used to immediately withdraw the embolic protection device for intravascular use by pulling the retract wire if the device does not achieve the expected result or if there is a problem after implantation.

The present disclosure also provides an embolic protection device for intravascular use, which is a spiral line formed by a flat superelastic metal wire including a fixing section. The fixing section secures the embolic protection device for intravascular use to the blood vessel wall by compression stress between metal wire and blood vessel, and the diameter of the central intercepting section is smaller than the minimum diameter of the target thrombus to be intercepted.

The beneficial effects of the present disclosure include:

The performance of the embolic protection device for intravascular enhance the ability to intercept smaller thrombi.

It can also improve the blood flow dynamics environment of the embolic protection device for intravascular use in the body, reduce the occurrence rate of turbulence, and further reduce the probability of thrombus formation.

In manufacturing, it reduces the difficulty of production and increases the efficiency of producing the embolic protection device for intravascular use.

### Brief Description of the drawings

Fig. 1 is a structural diagram of an embodiment of the present disclosure.
Fig. 2 is a structural diagram of another embodiment of the present disclosure.
Fig. 3 is an axial view of the morphology of the embolic protection device for intravascular use implanted in a blood vessel.

### Detailed Description of some of the embodiments

The present disclosure pertains to an embolic protection device for intravascular use, formed from a metal wire or strip with an elliptical or flat cross-section, preformed into a coaxial spiral or vortex configuration.

The formed embolic protection device for intravascular use can be deployed in a tubular object and introduced into the target blood vessel through the tubular object. The material's elasticity can recover to the expected shape to achieve the purpose of intercepting thrombi. Coaxial spiral lines can be manufactured, so the impact on the blood flow field is smaller compared to non-coaxial spiral lines, resulting in a more stable flow field and a smaller curvature of the intercepting area, ensuring intercepting performance.

Detailed description of the present disclosure is shown as following:

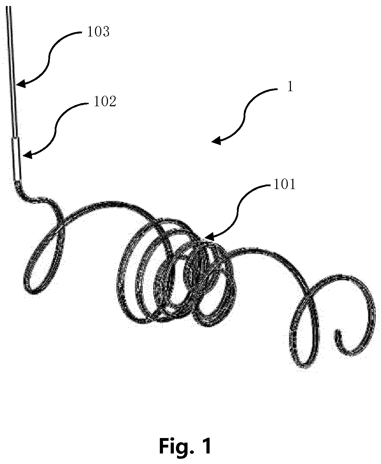

Reference is now made to Fig.1, which is a structural diagram of an embodiment of the present disclosure. As shown in Fig. 1, an embodiment of the present disclosure is a curve formed by a single wire winding. The embolic protection device 101 is characterized by a spiral line formed by a flat superelastic metal wire, with the axis of the spiral line forming an acute angle with the wide face of the flat wire. This embodiment can minimize the strain of the final embolic protection device 101, ensuring that the metal wire can recover its original shape after passing through a long straight channel. On the other hand, the width of the flat wire along axial direction is greater than thickness of the flat wire (usually more than 1.5 times the thickness of the flat wire), which minimizes the deformation of the embolic protection device 101 when subjected to blood flow impact, thereby avoiding blood flow deformation that disrupts blood flow. On the other hand, the thinner thickness side of the flat wire provides greater local stress to the accumulated thrombi on the embolic protection device 101 under blood flushing, thereby cutting soft thrombi into smaller pieces and avoiding local thrombus blockage. The connecting ring 102 is preferably made of a bio-degradable metal material, such as magnesium alloy, iron alloy, etc., or a biocompatible non-degradable material, such as titanium alloy, stainless steel, etc. Its main function is to connect the embolic protection device 101 with the retract wire 103. The connecting ring 102 can remain on the blood vessel wall after implantation, during which it will become endothelialized, and the endothelialization rate is greater than its degradation rate. The retract wire 103 is designed to immediately withdraw the embolic protection device for intravascular use by pulling the retract wire 103 if the device does not achieve the expected result or if there is a problem after implantation. The retract wire 103 is made of absorbable suture material that can withstand tensile force.

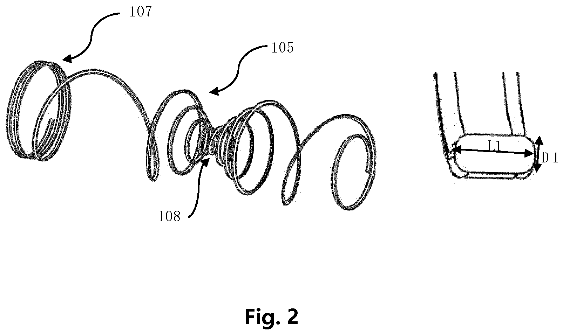

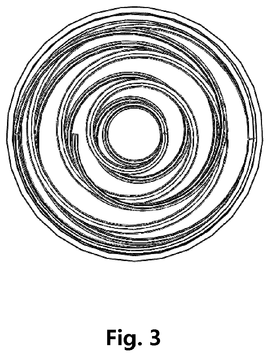

Fig. 2 shows the structural diagram of another embodiment of the present disclosure. The embolic protection device 105 is composed of a single superelastic metal wire, characterized by a spiral line formed by a flat superelastic metal wire. The fixing section 107 secures the embolic protection device for intravascular use to the blood vessel wall by compression stress between metal wire and blood vessel, and the diameter of the central intercepting section 108 is smaller than the minimum diameter of the target thrombus to be intercepted. Compared with embodiment one, the advantage is that there is no foreign object spanning the blood vessel wall after implantation, even though the foreign object in the previous embodiment will eventually degrade and become endothelialized. Fig. 3 shows the axial view of the morphology of the embolic protection device for intravascular use implanted in a blood vessel, with a coaxial void in the center, the diameter of which should be smaller than the minimum diameter of the target thrombus to be intercepted. It can also be seen that the distribution of the embolic protection device for intravascular use expands around the axis, with more uniform spacing.

Finite element analysis was used to simulate the embodiment of different cross-sections of the embolic protection device for intravascular use on the pressure on the blood vessel wall and the impact on blood flow. The simulation was based on the blood vessel wall in the systolic pressure (high pressure) state, where the inner diameter is 10% smaller than the outer diameter of the embolic protection device for intravascular use, with systolic pressure at 120 mmHg and diastolic pressure at 80 mmHg. The pressure and velocity changes of major branch arteries (carotid arteries, cerebral arteries) were used as boundary conditions, and the results are as follows:

For an embolic protection device for intravascular use with a circular cross-section of 0.25 mm in diameter, the maximum instantaneous pressure on the blood vessel wall is 52.3±7.8 KPa.

The local pressure difference before and after the embolic protection device for intravascular use with a circular cross-section of 0.25 mm in diameter is approximately 7.5 mmHg.

For an embolic protection device for intravascular use with an elliptical cross-section of 0.25 mm x 0.12 mm, the maximum instantaneous pressure on the blood vessel wall is 27.6±4.0 KPa.

The local pressure difference before and after the embolic protection device for intravascular use with an elliptical cross-section is approximately 5.7 mmHg.

Compared with a circular cross-section, an elliptical cross-section exerts less local pressure on the blood vessel wall, making it less likely to cause hyperplasia in the blood vessel wall. On the other hand, the pressure drop of blood flow through an elliptical cross-section is much lower than that of a circular cross-section, which can better maintain the pressure and velocity of blood flow in different sections and has a smaller impact on local blood flow.

Both coaxial and non-coaxial spiral line devices for obstructing thrombi for intravascular use did not show any reflux blood vectors in the blood flow field distribution (i.e., no local vortex phenomenon). The difference between the maximum and minimum local blood flow velocities during systole for the coaxial spiral line device and the non-coaxial spiral line device was 0.67 m/s and 1.32 m/s, respectively, with the maximum velocity in the central intercepting section and the minimum velocity in the rear section of the blood flow passing through the embolic protection device for intravascular use. Therefore, the coaxial spiral line embolic protection device for intravascular use has a smaller impact on local blood flow compared to the non-coaxial spiral line device and is more in line with the original human blood flow.

Technical personnel in this field can still make other deformations within the creative spirit of this disclosure. These various deformations derived based on the creative spirit of this disclosure should still be within the scope of protection of this disclosure.

## Claims

1. An embolic protection device for intravascular use, comprising:
a metal wire or strip with an elliptical or flat cross-section, preformed into a coaxial multi-turn spiral or vortex configuration.

2. The device of claim 1, the single wire can become a straight line in a constrained state and will recover to the preset shape in a free state.

3. The device of claim 1, the cross-section of the single wire is flat or elliptical.

4. The device of claim 1, the formed embolic protection device for intravascular use can be deployed in a tubular object, and can be introduced into the target blood vessel through the tubular object. The material's elasticity can recover to the expected shape to achieve the purpose of intercepting thrombi.

5. An embolic protection device for intravascular use, comprising:
a spiral line formed from a flat superelastic metal wire;
a connecting ring connecting the embolic protection device with a retract wire; and a retract wire designed to withdraw the embolic protection device if implantation does not achieve the expected result or in the case of failure after deployment.

6. The device of claim 5, the connecting ring can be endothelialized, and the endothelialization rate is greater than its degradation rate.

7. An embolic protection device for intravascular use, comprising:
a spiral line formed by a flat superelastic metal wire including a fixing section.
The fixing section secures the embolic protection device for intravascular use to the blood vessel wall by compression stress between metal wire and the blood vessel, and
the diameter of the central intercepting section is smaller than the minimum diameter of the target thrombus to be intercepted.
